# EUROPÄISCHE PATENTSCHRIFT

(11) **EP 3 781 541 B1**
(45) Veröffentlichungstag und Bekanntmachung des Hinweises auf die Patenterteilung: **27.04.2022**
(21) Anmeldenummer: 19714682.2
(22) Anmeldetag: 08.04.2019
(51) Int. Cl.: C07C 2/78, C01B 3/36, C07C 11/24

(54) **VERFAHREN ZUR HERSTELLUNG VON ACETYLEN UND SYNTHESEGAS**
METHOD FOR PRODUCING ACETYLENE AND SYNGAS
PROCÉDÉ DE PRODUCTION D'ACÉTYLÈNE ET DE GAZ DE SYNTHÈSE

(30) Priorität: 17.04.2018 EP 18167720
(43) Veröffentlichungstag der Anmeldung: 24.02.2021
(73) Patentinhaber: BASF SE, 67056 Ludwigshafen am Rhein (DE)
(72) Erfinder: VICARI, Maximilian, 67056 Ludwigshafen (DE); WEICHERT, Christian, 67056 Ludwigshafen (DE); ANDERLOHR, Christopher Alec, 67056 Ludwigshafen (DE); REIF, Wolfgang, 67056 Ludwigshafen (DE)
(74) Vertreter: BASF IP Association
(86) Internationale Anmeldenummer: PCT/EP2019/058740
(87) Internationale Veröffentlichungsnummer: WO 2019/201632

(56) Entgegenhaltungen:
- WO-A1-2013/186291
- WO-A1-2014/111396

## Beschreibung

Die vorliegende Erfindung betrifft ein Verfahren zur Herstellung von Acetylen und Synthesegas durch partielle Oxidation von Kohlenwasserstoffen mit Sauerstoff.

Die obige partielle Oxidation ist eine Hochtemperaturreaktion, die üblicherweise in einem Reaktorsystem, umfassend eine Mischeinrichtung, einen Brennerblock sowie eine Quencheinrichtung, durchgeführt wird, und beispielsweise in Ullmanns Encyclopedia of Industrial Chemistry (5th Edition, Volume A1, Seiten 97 - 144) oder US 005824834A beschrieben ist.

Gemäß Ullmanns Encyclopedia of Industrial Chemistry (5th Edition, Volume A1 Seiten 97 - 144) erfolgt die Aufheizung der Einsatzstoffe getrennt in Vorheizern. Die aufgeheizten Einsatzstoffe werden in einer Mischeinrichtung gemischt und über einen Mischdiffusor einem Brenner und weiter einem Feuerraum zugeführt. Stromab des Feuerraums wird mittels Düsen ein wässriges Quenchmedium dem Spaltgas zugeführt und dieses schnell auf etwa 80 - 90 °C abgekühlt. Der Prozess wird durch geeignete Wahl der Sauerstoffzahl λ so betrieben (λ < 0,31), wobei man unter der Sauerstoffzahl λ das Verhältnis aus der tatsächlich im zweiten Einsatzstrom vorhandenen Sauerstoffmenge zur stöchiometrisch notwendigen Sauerstoffmenge versteht, dass die Ausbeute an Acetylen bezogen auf das trockene Spaltgas optimal groß wird (> 8 %). Hierbei wird unter Sauerstoffzahl λ wie üblich das Verhältnis aus der tatsächlich vorhandenen Sauerstoffmenge zur stöchiometrisch notwendigen Stauerstoffmenge verstanden, die für die vollständige Verbrennung der Einsatzstoffe erforderlich ist. Hierbei wird jedoch auch die Rußbeladung des Spaltgases maximal. Der im Feuerraum aus der Gasphase gebildete Ruß wird zum Teil durch den Quench, in einer anschließenden Kühlkolonne und einem daran anschließenden Elektrofilter abgeschieden. Der wertprodukthaltige Produktgasstrom wird getrennt über die Kühlkolonne abgeführt. Nach dem Elektrofilter ist die Rußkonzentration im restlichen Spaltgas (ohne Wertprodukte) auf etwa 1 mg/m³ gesunken. Der im Prozesswasser aus dem Quench, der Kühlkolonne und dem Elektrofilter enthaltene Ruß besitzt einen hohen Kohlenwasserstoffanteil und ist daher hydrophob, was ihn auf dem Prozesswasser aufschwimmen lässt. Daher wird dieses rußbeladene Prozesswasser über sogenannte offene Rußrinnen mit Oberflächen-Partikel-Abscheidern geleitet. Die aufschwimmenden Rußanteile werden dabei abgetrennt und einer Feuerung zugeführt. Das so gereinigte Prozesswasser wird anschließend über einen offenen Kühlturm gefahren und somit abgekühlt. Dabei und während der Fest-Flüssigtrennung zuvor wird ein Großteil der flüssig und gasförmig im Prozesswasser gebundenen Kohlenwasserstoffe, insbesondere Aromate, Alkine, Benzol-Toluol-Xylol, etc. zusammen mit Teilen des Prozesswassers in die Umgebungsluft emittiert. Anschließend wird der so entstandene Verlust an Prozesswasser durch Zugabe kompensiert und der Wasserkreislauf Richtung Kühlkolonne und Quench geschlossen.

Die Emissionen an Kohlenwasserstoffen aus dem Prozesswasser aus dem Kühlturm (d.h. bei einer offenen Prozesswasserfahrweise) sind jedoch unter den geltenden Umweltschutzauflagen nicht mehr tragbar. Bei einer geschlossenen Prozesswasserfahrweise würden sich aber die Kohlenwasserstoffe anreichern und zu Polymerisation und Verstopfung der Anlage führen, so dass auch eine geschlossene Prozesswasserfahrweise keine tragbare Lösung ist. Eine weitere Emissionsquelle stellen die offenen Rußrinnen dar.

Ein weiteres Verfahren zur Herstellung von Acetylen und Synthesegas durch partielle Oxidation von Kohlenwasserstoffen mit Sauerstoff ist in US 005824834A beschrieben. Dabei handelt es sich um ein Rußmengen-optimiertes, geschlossenes Wasserquenchverfahren, das mit einem mageren Feedstrom betrieben wird, und zwar mit einem Feedstrom mit einer Sauerstoffzahl λ > 0,31. Das Verfahren hat jedoch den Nachteil einer reduzierten Ausbeute an Wertprodukt Acetylen.

Bei dieser Verfahrensvariante wird das wässrige Quenchmedium ebenfalls mittels Düsen das dem Spaltgas zugeführt und dieses schnell auf etwa 80 -90 °C abgekühlt. Der im Feuerraum aus der Gasphase gebildete Ruß wird zum Teil durch den Quench, eine anschließende mit rezirkulierendem Wasser betriebene Kühlkolonne und einem daran anschließenden Elektrofilter abgeschieden. Der werthaltige Produktgasstrom wird getrennt über die Kühlkolonne abgeführt. Der Prozess wird hierbei durch Wahl der Sauerstoffzahl λ so betrieben (λ > 0,31), dass die anfallende Rußmenge im Spaltgas so gering ist, dass alleine durch die Ausschleusung des anfallenden Reaktionswassers aus der Verbrennung der stationäre Betrieb gewährleistet werden kann. Dadurch reduziert sich jedoch der Acetylengehalt im trockenen Spaltgas um 2 Prozentpunkte gegenüber dem vorstehend beschriebenen Verfahren auf etwa 6 Vol.-%. Damit wird eine geschlossene, also gegenüber der Umwelt abgetrennte Wasserquench-Fahrweise ermöglicht. Der Vorteil gegenüber der zuvor beschriebenen Verfahrensvariante ist somit die Möglichkeit des geschlossenen Betriebes ohne weitere Trennapparaturen. Der Nachteil sind Ausbeuteeinbußen bezüglich des Wert- und Zielprodukts Acetylen.

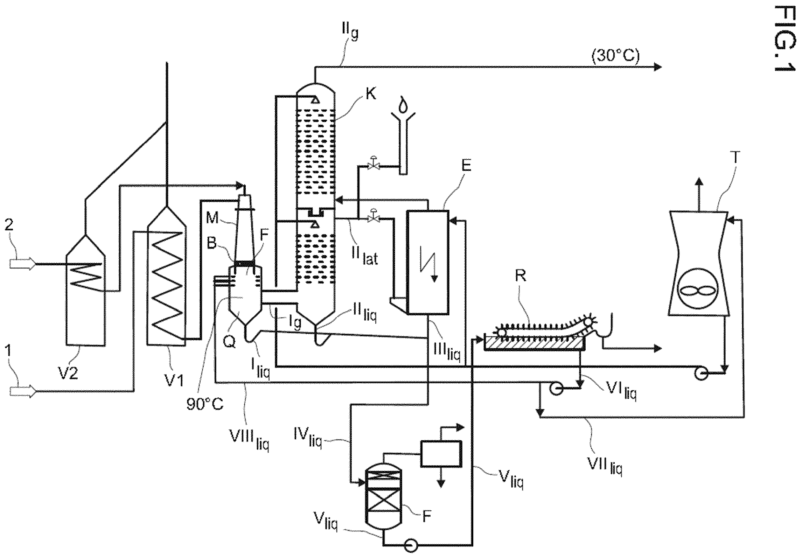

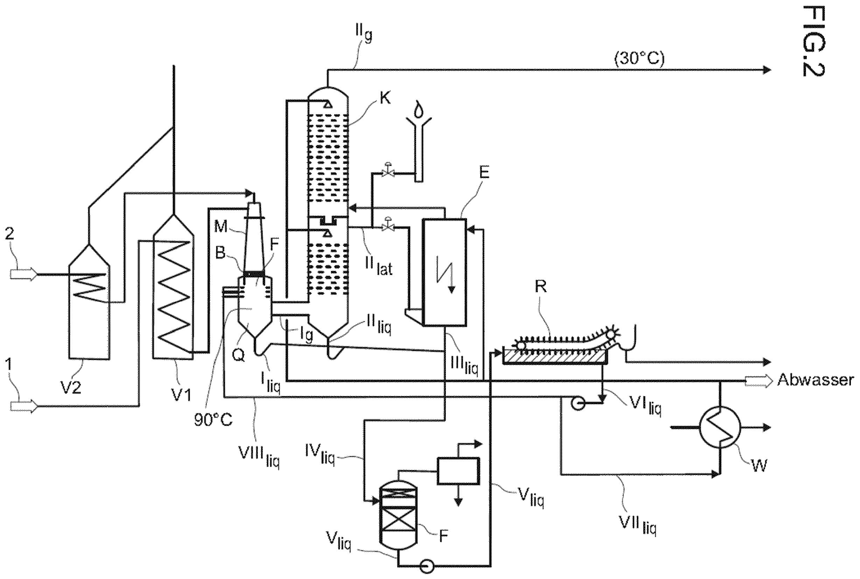

In der EP 2 861 527 B1 wird ein Verfahren zur Herstellung von Acetylen und Synthesegas durch partielle Oxidation von Kohlenwasserstoffen offenbart, welches die Vorteile der vorstehend genannten Verfahren kombiniert und somit eine hohe Ausbeute an Wertprodukt Acetylen ermöglicht als auch die unerwünschte Emission von Schadstoffen stark verringert. Hierbei werden vorteilhafterweise die unterwünschten, im Prozesswasserstrom gelösten Gase, welche bei unkontrolliertem Entweichen zu Umweltbelastungen führen können, in dem Entspannungsbehälter über die Gasphase abgeführt (Seite 3, Zeile 26 bis 33), wodurch das beschrieben Verfahren eine hohe Umweltverträglichkeit bietet. Hierbei wird der Prozesswasserstrom zuvor über die Rußrinnen geleitet (Figur 1 und Figur 2). Da der Prozesswasserstrom zu diesem Zeitpunkt noch die vorstehend beschriebenen unerwünschten Gase in gelöster Form enthält ist es erforderlich, die Rußrinnen aufwendig abzudichten, wodurch die Effektivität des Verfahrens beeinträchtigt wird.

Es stellte sich somit die Aufgabe, ein Verfahren zur Herstellung von Acetylen und Synthesegas durch partielle Oxidation von Kohlenwasserstoffen zur Verfügung zu stellen, welches weiterhin die Vorteile des in der EP 2 861 527 B1 beschriebenen Verfahrens gewährleistet und darüber hinaus die unerwünschte Emission von Schadstoffen in hoher Wirksamkeit effektiv und verfahrenstechnisch einfach sicherstellt.

Die Aufgabe wird gelöst durch ein Verfahren zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter, Sauerstoff enthaltender Einsatzstrom
- getrennt voneinander vorgeheizt,
in einem Verhältnis der Massenströme des zweiten Einsatzstromes zum ersten Einsatzstrom entsprechend einer Sauerstoffzahl λ von kleiner oder gleich 0,31 gemischt werden, die für die vollständige Verbrennung des einen oder der mehreren, im ersten Einsatzstrom enthaltenen Kohlenwasserstoffe, erforderlich ist,
- über einen Brennerblock einem Feuerraum zugeführt werden, worin die partielle Oxidation der Kohlenwasserstoffe stattfindet,
- unter Erhalt eines Spaltgases, das stromabwärts des Feuerraumes durch Eindüsen eines wässrigen Quenchmediums auf 80 bis 90 °C gequencht wird, wobei
   - ein Prozesswassersstrom I_{liq}, sowie
   - ein Produktgasstrom I_{g} erhalten wird, der
   - in einer Kühlkolonne durch direkten Wärmetausch mit Kühlwasser abgekühlt wird, unter Erhalt
   - eines Prozesswasserstromes II_{liq} als Sumpfstrom,
   - eines Produktgasstromes II_{g} als Kopfstrom, sowie
   - eines Seitenstromes IIₗₐₜ, der
- in einem Elektrofilter an Ruß abgereichert wird und wobei im Elektrofilter ein Prozesswasserstrom II_{liq} anfällt, welcher mit den Prozesswasserströmen I_{liq} und II_{liq} vereinigt wird zu dem Prozesswasserstrom IV_{liq}, welches dadurch gekennzeichnet ist, dass der vereinigte Prozesswasserstrom IV_{liq}einer Reinigung durch Teilverdampfung in einem Entspannungsbehälter unterworfen wird, wobei der vereinigte Prozesswasserstrom IV_{liq} zu einem Anteil von 0,01 Gew.-% bis 10 Gew.-% bezogen auf das Gesamtgewicht desselben, verdampft wird, unter Erhalt eines gereinigten Prozesswasserstromes V_{liq} der am Sumpf des Entspannungsbehälters abgezogen wird und über Rußrinnen mit Oberflächen-Partikelabscheidern geleitet wird, unter Erhalt eines von aufschwimmenden Russ befreiten Prozesswasserstromes VI_{liq}, der in das Verfahren recycliert wird.

Es wurde gefunden, dass durch eine Teilverdampfung der vereinigten Prozesswasserströme in einem Entspannungsbehälter die unerwünschten gelösten Gase, insbesondere polymerisierbare Komponenten, beispielsweise höhere Acetylene, aus den Prozesswasserströmen mit dem Entspannungsdampf in die Gasphase mitgerissen werden, und soweit von der Flüssigphase, dem vereinigten Prozesswasserstrom, abgetrennt werden können, dass dieser in das Verfahren recycliert von welchem auch das überschüssige, anfallende Abwasser entsorgt werden kann.

Durch die Einbindung des Entspannungsbehälters vor den Rußrinnen wird vorteilhafterweise sichergestellt, dass die unerwünschten gelösten Gase frühzeitig effektiv aus dem Prozesswasserstrom abgezogen werden. Hierdurch können aufwendige Abdichtungsmaßnahmen resultierend von potentiellen Emissionen solcher unerwünschter gelösten Gase bei den nachgeschalteten Rußrinnen vorteilhafterweise vermieden werden und das Verfahren insgesamt effektiver und wirtschaftlicher gestaltet werden.

Die mit dem Entspannungsdampf mitgerissenen Dämpfe von unerwünschten gelösten Gasen können anschließend zum Beispiel nach Kondensation des Wasserdampfes verbrannt oder anderweitig im Prozess entsorgt werden.

Es hat sich überraschend gezeigt, dass eine einstufige Entspannung zur Teilverdampfung des vereinigten Prozesswasserstromes, zu einem Anteil von 0,01 bis 10 Gew.-%, bezogen auf das Gesamtgewicht des vereinigten Prozesswasserstromes eine ausreichende Abreicherung an unerwünschten gelösten Komponenten ermöglicht, so dass das Verfahren in einem geschlossenen Prozesswasserkreislauf betrieben werden kann.

In einer bevorzugten Ausführungsform wird das Verfahren mit einem geschlossenen Prozesswasserkreislauf betrieben. In dieser Verfahrensvariante wird bevorzugt ein Teilstrom des gereinigten Prozesswasserstromes aus dem Verfahren ausgeschleust und der übrige Teilstrom des gereinigten Prozesswasserstromes in das Verfahren recycliert. Bevorzugt wird hierbei der die Rußrinnen verlassende Prozesswasserstrom VI_{liq} aufgeteilt und ein Teilstrom dieses Prozesswasserstromes VI_{liq} als Prozesswasserstrom VII_{liq} einem Wärmetauscher zugeführt und nach Abkühlung ein Teilstrom dieses gekühlten Prozesswasserstromes in die Kühlkolonne zurückgeführt und der restliche Teilstrom in das Abwasser entsorgt und der zweite Teilstrom des die Rußrinnen verlassenden Prozesswasserstromes VI_{liq} als Prozesswasserstrom VIII_{liq} in den Quenschbereich unterhalb des Brennerblocks zurückgeführt.

In einer weiteren bevorzugten Verfahrensvariante wird der die Rußrinnen verlassende Prozesswasserstrom VI_{liq} aufgeteilt und ein Teilstrom dieses Prozesswasserstromes VI_{liq} wird als Prozesswasserstrom VII_{liq} einem Kühlturm zugeführt und dort abgekühlt und anschließend in die Kühkolonne zurückgeführt und der zweite Teilstrom des Prozesswasserstromes VI_{liq} wird als Prozesswasserstrom VIII_{liq} in den Quenschbereich unterhalb des Brennerblocks zurückgeführt. In dieser Verfahrensvariante wird bevorzugt der gesamte gereinigte Prozesswasserstrom in das Verfahren recycliert.

Bevorzugt wird der vereinigte Prozesswasserstrom zu einem Anteil von 0,5 Gew.-% bis 5 Gew.-%, bezogen auf das Gesamtgewicht desselben, verdampft.

Das Verfahren zur Herstellung von Acetylen und Synthesegas wird erfindungsgemäß mit einer Sauerstoffzahl λ von kleiner oder gleich 0,31 betrieben, wobei unter Sauerstoffzahl λ das Verhältnis aus der tatsächlich im zweiten Einsatzstrom vorhandenen Sauerstoffmenge zur stöchiometrisch notwendigen Sauerstoffmenge verstanden wird, die für die vollständige Verbrennung des einen oder der mehreren, im ersten Einsatzstrom enthaltenen Kohlenwasserstoffe erforderlich ist.

Bei einem Betrieb mit einer Sauerstoffzahl λ im obigen Bereich wird eine hohe Ausbeute an Wertprodukt Acetylen gewährleistet.

Das Verfahren ist unabhängig von der konkreten Ausbildung des Reaktorsystems umfassend Mischeinrichtung, Brennerblock sowie die Quencheinrichtung.

Im Folgenden werden die üblicherweise eingesetzten Reaktorsysteme näher erläutert:
Die Ausgangsstoffe, das heißt ein Kohlenwasserstoff enthaltender Gasstrom, insbesondere Erdgas, und Sauerstoff, werden dabei getrennt aufgeheizt, üblicherweise bis hin zu 600 °C. In einer Mischeinrichtung werden die Reaktanden intensiv vermischt und nach Durchströmen eines Brennerblocks zur exothermen Reaktion gebracht. Der Brennerblock besteht üblicherweise aus einer Vielzahl von parallelen Kanälen, in denen die Strömungsgeschwindigkeit der zündfähigen Sauerstoff/Kohlenwasserstoff-Mischung höher ist als die Flammengeschwindigkeit, um ein Durchschlagen der Flamme in die Mischeinrichtung zu verhindern. Der metallische Brennerblock wird gekühlt, um den thermischen Belastungen standzuhalten. Je nach Aufenthaltszeit in der Mischeinrichtung besteht die Gefahr der Vor- und Rückzündung aufgrund der begrenzten thermischen Stabilität der Mischungen. Hierzu wird der Begriff der Zündverzugszeit, bzw. Induktionszeit gebraucht als die Zeitspanne, in der eine zündfähige Mischung keine nennenswerte intrinsische thermische Veränderung durchläuft. Die Induktionszeit ist abhängig von der Art der eingesetzten Kohlenwasserstoffe, dem Mischungszustand, von Druck und Temperatur. Sie bestimmt die maximale Aufenthaltszeit der Reaktanden in der Mischeinrichtung. Reaktanden wie Wasserstoff, Flüssiggas oder Leichtbenzin, deren Einsatz aufgrund von Ausbeute- und /oder Kapazitätssteigerungen im Syntheseprozess besonders wünschenswert ist, zeichnen sich durch eine vergleichsweise hohe Reaktivität und damit geringe Induktionszeit aus.

Die im derzeitigen Produktionsmaßstab eingesetzten Acetylenbrenner zeichnen sich durch die zylinderförmige Geometrie des Feuerraums aus. Der Brennerblock weist vorzugsweise hexagonal angeordnete Durchführungsbohrungen auf. In einer Ausführungsform sind z.B. 127 Bohrungen á 27 mm Innendurchmesser hexagonal auf einem kreisförmigen Grundquerschnitt mit Durchmesser von ca. 500 mm angeordnet. In der Regel liegen die eingesetzten Kanaldurchmesser bei etwa 19 bis 27 mm Durchmesser. Der anschließende Feuerraum, in dem die Flamme der acetylenbildenden partiellen Oxidationsreaktion stabilisiert wird, ist üblicherweise ebenfalls von zylindrischem Querschnitt, ist wassergekühlt und entspricht im Erscheinungsbild dem eines kurzen Rohres (von z.B. 180 bis 533 mm Durchmesser und 380 bis 450 mm Länge).

In Höhe des Brennerblocks wird sowohl in axialer als auch in radialer Richtung sogenannter Hilfssauerstoff dem Feuerraum zugeführt. Dadurch wird für eine Flammenstabilisierung und somit für einen definierten Abstand der Flammenwurzel und damit des Reaktionsbeginns zum Reaktionsabbruch durch die Quencheinrichtung gesorgt. Der gesamte Brenner aus Brennerblock und Feuerraum wird in einen Quenchbehälter größeren Querschnitts über einen Flansch von oben eingehängt. Auf Höhe der Austrittsebene aus dem Feuerraum sind am äußeren Umfang derselben Quenchdüsen auf einem oder mehreren Quenchverteilerringen installiert, die das Quenchmedium mit oder ohne Zuhilfenahme eines Zerstäubungsmedium zerstäuben und näherungsweise senkrecht zur Hauptströmungsrichtung der den Feuerraum verlassenden Reaktionsgase eindüsen. Dieser direkte Quench hat die Aufgabe das Reaktionsgemisch extrem schnell abzukühlen, so dass Folgereaktionen, d.h. insbesondere der Abbau von gebildetem Acetylen, eingefroren werden. Die Reichweite und Verteilung der Quenchstrahlen ist dabei idealerweise so bemessen, dass eine möglichst homogene Temperaturverteilung in möglichst kurzer Zeit erreicht wird.

Bei dem vorliegenden technischen Verfahren entsteht neben Acetylen im Wesentlichen Wasserstoff, Kohlenmonoxid und Ruß. Die in der Flammenfront gebildeten Rußpartikel können als Keime an den Feuerraum-Seitenwänden anhaften, worauf es bei geeigneten physikalisch-chemischen Bedingungen zum Aufwachsen, Ablagern und Anbacken von Koksschichten kommt. Diese Ablagerungen werden periodisch im Bereich der Feuerraumwände mittels einer Stocherreinrichtung mechanisch abgereinigt.

Die vorliegende Erfindung nutzt den Umstand, dass beim obigen Wasserquenchverfahren ein Prozesswasserstrom I_{liq} (ein Quenchwasser) bei einer Temperatur im Bereich zwischen 60 und 96°C, bevorzugt mit einer Temperatur im Bereich von circa 70 bis 90°C, anfällt. Die enthaltene thermische Energie erlaubt eine ausreichende Abtrennung von unerwünschten gelösten Gasen durch Teilverdampfung ins Vakuum.

Bevorzugt erfolgt die Teilverdampfung durch einstufige Entspannung ins Vakuum.

Weiter bevorzugt erfolgt die Teilverdampfung durch einstufige Entspannung adiabat.

In einer Verfahrensvariante kann die Teilverdampfung vorteilhaft durch Wärmeeintrag unterstützt werden.

Eine hinreichende Abtrennung der gelösten Gase kann auch durch eine Strippkolonne erreicht werden. Hierzu wird der vereinigte Prozesswasserstrom auf den Kopf der Kolonne und der Strippdampf im Gegenstrom in den Sumpf der Strippkolonne gegeben. Auch mit diesem Verfahrensschritt wird eine ausreichende Abreicherung der gelösten Gase erreicht. Der apparative Aufwand und somit auch die Investitionskosten des verfahrenstechnischen Schritts sind deutlich höher als beim einfachen, erfindungsgemäßen Flash. Außerdem neigen die Einbauten der dann notwendigen Trennstufen und Verteiler deutlich mehr zum Verschmutzen durch polymerisierende Komponenten als der einfache Aufbau einer einstufigen Entspannung.

Der Entspannungsbehälter ist bevorzugt einstufig und kann mit üblichen Einbauten, wie Packungen oder Böden, wie auch mit einem Demister gegen Tröpfchenmitriss ausgestattet sein.

Möglich ist auch eine mehrstufige Entspannung oder ein Wärmeeintrag im Sumpf wie bei einer Destillationskolonne statt Vorheizen des Zulaufs.

Somit stellt dieses Verfahren eine sehr kostengünstige Möglichkeit der Kreislaufwasserreinigung, bzw. Abwasserreinigung dar.

Das Vakuum kann nach im Stand der Technik bekannter Weise z. B. über eine Dampfstrahlanlage oder einen Wasserringverdichter erzeugt werden. Das Abgas lässt sich dann innerhalb der Anlage weiter behandeln oder auch einer Abgasverbrennung zuführen.

Die Erfindung wird im Folgenden anhand einer Zeichnung sowie in Ausführungsbeispielen näher erläutert.

In der Zeichnung zeigen im Einzelnen:
Figur 1 die schematische Darstellung einer bevorzugten erfindungsgemäßen Anlage mit Kühlturm und Figur 2 die schematische Darstellung einer weiteren bevorzugten Anlage zur Durchführung des erfindungsgemäßen Verfahrens ohne Kühlturm.

Der in Figur 1 dargestellten Anlage wird ein Kohlenwasserstoff enthaltender Gasstrom (1) sowie ein Sauerstoff enthaltender Gasstrom (2) zugeführt, über Vorheizer V1 bzw. V2, voneinander getrennt vorgeheizt, in einer Mischeinrichtung (M) gemischt, über einen Brennerblock (B) einem Feuerraum (F) zugeführt und anschließend in einem Quenchbereich (Q) durch Eindüsen eines wässrigen Quenchmediums gequencht, wobei ein Prozesswasserstrom I_{liq} sowie ein Produktgasstrom I_{g} erhalten werden.

Der Produktgasstrom I_{g} wird in einer Kühlkolonne (K) durch direkten Wärmetausch mit Kühlwasser abgekühlt, unter Erhalt eines Prozesswasserstromes II_{liq} als Sumpfstrom, eines Produktgasstromes II_{g} als Kopfstrom sowie eines Seitenstromes IIₗₐₜ. Der Seitenstrom IIₗₐₜ wird einem Elektrofilter (E) zugeführt und dort an Ruß abgereichert, wobei ein Prozesswasserstrom III_{liq} entsteht. Am Kopf des Elektrofilters wird das gereinigte Gas abgeleitet und der Kühlkolonne zugeführt. Von dem die Kühlkonne verlassenden Seitenstrom IIₗₐₜ kann bei Bedarf (Anfahren der Anlage, Störungen) ein Strom auf eine Spaltgasfackel geführt werden. Die Prozesswasserströme I_{liq}, II_{liq} und II_{liq} werden zu Prozesswasserstrom IV_{liq} vereinigt einem einstufigen Entspannungsbehälter (F) zugeführt und darin teilverdampft, unter Erhalt eines gereinigten Prozesswasserstromes V_{liq}. Dieser gereinigte Prozesswasserstrom V_{liq} wird über die Rußrinnen (R) mit Oberflächen-Partikelabscheidern geleitet um den aufschwimmenden Ruß abzuscheiden. Am Kopf des Entspannungsbehälters wird der anfallende Flashdampf sowie innerte Bestandteile abgezogen und einer Vakuumanlage zugeführt. Der die Rußrinnen verlassende Prozesswasserstrom VI_{liq} wird aufgeteilt und ein Teilstrom dieses Prozesswasserstromes VI_{liq} wird als Prozesswasserstrom VII_{liq} einem Kühlturm (T) zugeführt und dort abgekühlt und anschließend in die Kühlkolonne (K) zurückgeführt. Ein Teilstrom dieses zurückgeführten Prozesswasserstroms wird in den oberen Bereich des Elektrofilters zur Reinigung der Drähte als Spülstrom zugeführt. Der zweite Teilstrom des Prozesswasserstromes VI_{liq} wird als Prozesswasserstrom VIII_{liq} in den Quenschbereich unterhalb des Brennerblocks zurückgeführt.

Die in Figur 2 dargestellte weitere bevorzugte Ausführungsform zeigt eine weitgehend analoge Anlage, wobei jedoch anstelle des Kühlturmes (T) ein Wärmetauscher (W) vorgesehen ist. Der die Rußrinnen verlassende Prozesswasserstrom VI_{liq} wird aufgeteilt und ein Teilstrom dieses Prozesswasserstromes VI_{liq} als Prozesswasserstrom VII_{liq} einem Wärmetauscher (W) zugeführt und nach Abkühlung ein Teilstrom dieses gekühlten Prozesswasserstromes in die Kühlkolonne (K) zurückgeführt und der restliche Teilstrom in das Abwasser entsorgt und der zweite Teilstrom des die Rußrinnen verlassenden Prozesswasserstromes VI_{liq} wird als Prozesswasserstrom VIII_{liq} in den Quenschbereich unterhalb des Brennerblocks zurückgeführt.

Ausführungsbeispiele:
Vergleichsbeispiel:
Ohne Prozesswasserreinigung ergeben sich in einer Anlage entsprechend der schematischen Darstellung in Figur 1 spezifisch für 1 t Acetylen folgende Emissionen aus den offenen Rußrinnen und der Abluft des Kühlturms:

| Emissionen offener Wasserquench | | | |
|---|---|---|---|
| | Rußrinnen kg/t Ac | Kühlturm kg/t Ac | gesamt kg/t Ac |
| CO | 0,303 | 0,486 | 0,789 |
| CH4 | 5,69E-02 | 1,05E-01 | 0,162 |
| C2H6 | 7,66E-03 | 1,47E-02 | 0,022 |
| C2H4 | 7,00E-03 | 2,85E-02 | 0,036 |
| C2H2 | 1,66E-01 | 5,31E+00 | 5,475 |
| PROPEN | 5,30E-04 | 1,91E-03 | 0,002 |
| PROPDIEN | 1,01E-03 | 3,65E-03 | 0,005 |
| PROPIN | 2,40E-03 | 8,59E-02 | 0,088 |
| BUTENIN | 1,73E-03 | 3,93E-02 | 0,041 |
| BUTADIIN | 7,58E-03 | 7,05E-01 | 0,712 |
| BENZOL | 2,40E-03 | 1,36E-01 | 0,138 |
| NAPHTLIN | 5,69E-04 | 1,09E-02 | 0,011 |

Ausführungsbeispiele nach der Erfindung:
Die Abreinigungseffizienz des Prozesswassers ist eine Funktion der Flashdampfmenge wie in der folgenden Tabelle dargestellt:
Dazu wird das Prozesswasser ausgehend von 87,3°C und 1,013 bar absolut auf Drücke zwischen 200 mbar absolut und 800 mbar absolut entspannt. Dabei wird das Prozesswasser zu einem Anteil von 0,0038 Gew. % bis 4,94 Gew. % teilverdampft. Es ergeben sich als Funktion des Entspannungsdrucks folgende Abreicherungen an gelösten Gasen.

| Abreicherung durch Flash in Abhängigkeit des Drucks (offener Wasserquench) | | | | |
|---|---|---|---|---|
| Temperatur Austritt [°C] | 87,3 | 85,7 | 75,8 | 60,1 |
| Temperatur Eintritt [°C] | 87,4 | 87,4 | 87,2 | 87,1 |
| Druck Eintritt [bar(absolut.)] | 1,013 | 1,013 | 1,013 | 1,013 |
| Druck Austritt [mbar(absolut)] | 800 | 600 | 400 | 200 |
| Flashdampfmenge bezogen auf Zulauf [%] | 0,0038% | 0,3108% | 2,14% | 4,94% |
| | Abreicherung | Abreicherung | Abreicherung | Abreicherung |
| CO | 87,7% | 99,9% | 99,99% | 100,00% |
| Methan | 84,8% | 99,8% | 99,98% | 100,00% |
| Ethan | 83,9% | 99,8% | 99,98% | 100,00% |
| Ethylen | 63,1% | 99,4% | 99,94% | 99,98% |
| Acetylen | 14,2% | 93,5% | 99,31% | 99,84% |
| Propen | 66,8% | 99,5% | 99,95% | 99,99% |
| Propadien | 66,8% | 99,5% | 99,95% | 99,99% |
| Propin | 12,8% | 92,7% | 99,15% | 99,77% |
| Butenin | 19,1% | 95,4% | 99,47% | 99,86% |
| Butadiin | 5,2% | 82,4% | 97,42% | 99,14% |
| Benzol | 8,4% | 88,9% | 98,66% | 99,62% |
| Naphthalin | 22,1% | 96,1% | 99,56% | 99,88% |

Es zeigt sich deutlich, dass die Abreicherung stark vom Entspannungsdruck abhängt. Erfolgt eine erfindungsgemäße, z.B. einstufige Entspannung des Prozesswassers vor dem Kühlturm, so ergeben sich nur mehr folgende Emissionen an die Umwelt:
Das Prozesswasser tritt mit 87.4°C in die einstufige Flash-Stufe ein und wird dabei auf 400 mbar absolut entspannt.

Dabei kühlt sich der Strom von 87.4°C auf 75.8°C ab und es entstehen dabei 2,14 % FlashDampf bezogen auf den Zulauf. In der Tabelle ist außerdem noch die Abreicherung durch den Reinigungsschritt in Prozenten angegeben

| Emissionen offener Wasserquench mit Flash | | |
|---|---|---|
| | Kühlturm kg/t | Abreicherung in % |
| CO | 1,05E-04 | 99,9866% |
| Methan | 2,87E-05 | 99,9823% |
| Ethan | 4,29E-06 | 99,9808% |
| Ethylen | 2,29E-05 | 99,9356% |
| Acetylen | 3,80E-02 | 99,3053% |
| Propen | 1,31E-06 | 99,9462% |
| Propadien | 2,50E-06 | 99,9463% |
| Propin | 7,54E-04 | 99,1462% |
| Butenin | 2,17E-04 | 99,4727% |
| Butadiin | 1,84E-02 | 97,4207% |
| Benzol | 1,84E-03 | 98,6649% |
| Naphthalin | 5,10E-05 | 99,5561% |

Aufgrund der hohen Abreicherungsrate kann der Kühlturm durch einen geschlossenen Wärmetauscher ersetzt werden, ohne dass es im Prozess zu nicht tolerablen Aufpegelungen von polymerisierbaren Komponenten, insbesondere von höheren Acetylenen und Naphthalin, kommt.

| | Nebenkomponenten im Prozesswasser | |
|---|---|---|
| | geschlossener Wasserquench ohne Flash [Gew.ppm] | geschlossener Wasserquench mit Flash [Gew.ppm] |
| CO | 2,367 | 0,001 |
| Methan | 0,511 | 0,000 |
| Ethan | 0,071 | 0,000 |
| Ethylen | 0,139 | 0,000 |
| Acetylen | 25,812 | 0,186 |
| Propen | 0,009 | 0,000 |
| Propadien | 0,018 | 0,000 |
| Propin | 0,417 | 0,004 |
| Butenin | 0,191 | 0,001 |
| Butadiin | 3,410 | 0,089 |
| Benzol | 0,018 | 0,009 |
| Naphthalin | 0,053 | 0,000 |

## Patentansprüche

1. Verfahren zur Herstellung von Acetylen und Synthesegas durch partielle Oxidation von Kohlenwasserstoffen mit Sauerstoff, wobei ein erster Einsatzstrom, enthaltend einen oder mehrere Kohlenwasserstoffe, und ein zweiter, Sauerstoff enthaltender Einsatzstrom
- getrennt voneinander vorgeheizt,
in einem Verhältnis der Massenströme des zweiten Einsatzstromes zum ersten Einsatzstrom entsprechend einer Sauerstoffzahl λ von kleiner oder gleich 0,31 gemischt werden, wobei man unter der Sauerstoffzahl λ das Verhältnis aus der tatsächlich im zweiten Einsatzstrom vorhandenen Sauerstoffmenge zur stöchiometrisch notwendigen Sauerstoffmenge versteht, die für die vollständige Verbrennung des einen oder der mehreren, im ersten Einsatzstrom enthaltenen Kohlenwasserstoffe, erforderlich ist,
- über einen Bennerblock einem Feuerraum zugeführt werden, worin die partielle Oxidation der Kohlenwasserstoffe stattfindet,
- unter Erhalt eines Spaltgases, das stromabwärts des Feuerraumes durch Eindüsen eines wässrigen Quenchmediums auf 80 bis 90 Grad Celsius gequencht wird, wobei
- ein Prozesswassersstrom I_{liq}, sowie
- ein Produktgasstrom I_{g} erhalten wird, der
- in einer Kühlkolonne durch direkten Wärmetausch mit Kühlwasser abgekühlt wird, unter Erhalt
- eines Prozesswasserstromes II_{liq} als Sumpfstrom,
- eines Produktgasstromes II_{g} als Kopfstrom, sowie
- eines Seitenstromes, der
- in einem Elektrofilter an Ruß abgereichert wird und wobei im Elektrofilter ein Prozesswasserstrom II_{liq} anfällt, welcher mit den Prozesswasserströmen I_{liq} und II_{liq} vereinigt wird zu dem Prozesswasserstrom IV_{liq}, **dadurch gekennzeichnet, dass** der vereinigte Prozesswasserstrom IV_{liq} einer Reinigung durch Teilverdampfung in einem Entspannungsbehälter unterworfen wird, wobei der vereinigte Prozesswasserstrom IV_{liq} zu einem Anteil von 0,01 Gew.-% bis 10 Gew.-% bezogen auf das Gesamtgewicht desselben, verdampft wird, unter Erhalt eines gereinigten Prozesswasserstromes V_{liq}, der am Sumpf des Entspannungsbehälters abgezogen wird und über Rußrinnen mit Oberflächen-Partikelabscheidern geleitet wird unter Erhalt eines von aufschwimmenden Ruß befreiten Prozesswasserstromes VI_{liq}, der in das Verfahren recycliert wird.

2. Verfahren nach Anspruch 1, **dadurch gekennzeichnet, dass** der gereinigte Prozesswasserstrom V_{liq} vollständig in das Verfahren recycliert wird.

3. Verfahren nach Anspruch 1, **dadurch gekennzeichnet, dass** der die Rußrinnen verlassende Prozesswasserstrom VI_{liq} aufgeteilt wird und ein Teilstrom dieses Prozesswasserstromes VI_{liq} als Prozesswasserstrom VII_{liq} einem Kühlturm zugeführt wird und dort abgekühlt wird und anschließend in die Kühkolonne zurückgeführt wird und der zweite Teilstrom des Prozesswasserstromes VI_{liq} als Prozesswasserstrom VIII_{liq} in den Quenschbereich unterhalb des Brennerblocks zurückgeführt wird.

4. Verfahren nach Anspruch 1, **dadurch gekennzeichnet, dass** der die Rußrinnen verlassende Prozesswasserstrom VI_{liq} aufgeteilt wird und ein Teilstrom dieses Prozesswasserstromes VI_{liq} als Prozesswasserstrom VII_{liq} einem Wärmetauscher zugeführt wird und nach Abkühlung ein Teilstrom dieses gekühlten Prozesswassertromes in die Kühlkolonne zurückgeführt wird und der restliche Teilstrom in das Abwasser entsorgt wird und der zweite Teilstrom des die Rußrinnen verlassenden Prozesswasserstromes VI_{liq} als Prozesswasserstrom VIII_{liq} in den Quenschbereich unterhalb des Brennerblocks zurückgeführt wird

5. Verfahren nach einem der Ansprüche 1 bis 4, **dadurch gekennzeichnet, dass** der vereinigte Prozesswasserstrom IV_{liq} zu einem Anteil von 0,5 Gew. % bis 5 Gew. %, bezogen auf das Gesamtgewicht desselben, verdampft wird.

6. Verfahren nach einem der Ansprüche 1 bis 5, **dadurch gekennzeichnet, dass** die Teilverdampfung durch Entspannung ins Vakuum erfolgt.

7. Verfahren nach einem der Ansprüche 1 bis 6, **dadurch gekennzeichnet, dass** die Teilverdampfung durch Entspannung in ein Vakuum von 50 bis 900 mbar a erfolgt.

8. Verfahren nach einem der Ansprüche 1 bis 7, **dadurch gekennzeichnet, dass** die Teilverdampfung durch Entspannung in ein Vakuum von 200 bis 600 mbar a erfolgt.

9. Verfahren nach einem der Ansprüche 1 bis 8, **dadurch gekennzeichnet, dass** die Teilverdampfung durch Entspannung adiabat erfolgt.

10. Verfahren nach einem der Ansprüche 1 bis 9, **dadurch gekennzeichnet, dass** die Teilverdampfung durch Wärmeeintrag unterstützt wird.

11. Verfahren nach Anspruch 10, **dadurch gekennzeichnet, dass** der Wärmeeintrag durch direkte Dampfeinspeisung erfolgt.

## Claims

1. A process for producing acetylene and synthesis gas by partial oxidation of hydrocarbons with oxygen, wherein a first input stream comprising one or more hydrocarbons and a second oxygen-comprising input stream
- are separately preheated,
mixed in a ratio of the mass flows of the second input stream to the first input stream corresponding to an oxygen number λ of not more than 0.31, wherein the oxygen number λ is understood as meaning the ratio of the oxygen amount actually present in the second input stream to the stoichiometrically necessary oxygen amount as required for complete combustion of the one or more hydrocarbons present in the first input stream,
- fed via a burner block to a combustion chamber in which the partial oxidation of the hydrocarbons is carried out
- to obtain a cracking gas which is quenched to 80 to 90 degrees Celsius downstream of the combustion chamber by injection of an aqueous quench medium to obtain
- a process water stream I_{liq} and
- a product gas stream I_{g} which
- is cooled in a cooling column by direct heat exchange with cooling water to obtain
- a process water stream II_{liq} as the bottom stream,
- a product gas stream II_{g} as the top stream and
- a sidestream which
- is depleted of soot in an electrofilter to generate in the electrofilter a process water stream III_{liq} which is combined with the process water streams I_{liq} and II_{liq} to afford the process water stream IV_{liq}, wherein the combined process water stream IV_{liq} is subjected to a purification by partial evaporation in a decompression vessel, wherein the combined process water stream IV_{liq} is evaporated in a proportion of 0.01% by weight to 10% by weight based on the total weight thereof to obtain a purified process water stream V_{liq} which is withdrawn at the bottom of the decompression vessel and passed through soot channels having surface particle separators to obtain a process water stream VI_{liq} freed of floating soot which is recycled into the process.

2. The process according to claim 1, wherein the purified process water stream V_{liq} is completely recycled into the process.

3. The process according to claim 1, wherein the process water stream VI_{liq} exiting the soot channels is divided up and a substream of this process water stream VI_{liq} is supplied as process water stream VII_{liq} to a cooling tower and cooled therein and subsequently recycled into the cooling column and the second substream of the process water stream VI_{liq} is recycled as process water stream VIII_{liq} into the quench region below the burner block.

4. The process according to claim 1, wherein the process water stream VI_{liq} exiting the soot channels is divided up and a substream of this process water stream VI_{liq} is supplied to a heat exchanger as process water stream VII_{liq} and, after cooling, a substream of this cooled process water stream is recycled into the cooling column and the remaining substream is discharged into the wastewater and the second substream of the process water stream VI_{liq} exiting the soot channels is recycled into the quench region below the burner block as process water stream VIII_{liq}.

5. The process according to any of claims 1 to 4, wherein the combined process water stream IV_{liq} is evaporated in a proportion of 0.5% to 5% by weight based on the total weight thereof.

6. The process according to any of claims 1 to 5, wherein the partial evaporation is carried out by decompression into vacuum.

7. The process according to any of claims 1 to 6, wherein the partial evaporation is carried out by decompression into a vacuum of 50 to 900 mbar a.

8. The process according to any of claims 1 to 7, wherein the partial evaporation is carried out by decompression into a vacuum of 200 to 600 mbar a.

9. The process according to any of claims 1 to 8, wherein the partial evaporation is carried out by adiabatic decompression.

10. The process according to any of claims 1 to 9, wherein the partial evaporation is assisted by heating.

11. The process according to claim 10, wherein the heating is carried out by direct steam injection.

## Revendications

1. Procédé pour la préparation d'acétylène et de gaz de synthèse par oxydation partielle d'hydrocarbures avec de l'oxygène, un premier flux d'alimentation contenant un ou plusieurs hydrocarbures et un deuxième flux d'alimentation contenant de l'oxygène
- étant préchauffés séparément l'un de l'autre, mélangés en un rapport des flux de masse du deuxième flux d'alimentation sur le premier flux d'alimentation correspondant à un indice d'oxygène À, inférieur ou égal à 0,31, l'indice d'oxygène À étant compris comme le rapport de la quantité réelle d'oxygène présente dans le deuxième flux d'alimentation sur la quantité d'oxygène stœchiométriquement nécessaire qui est requise pour la combustion totale du ou des hydrocarbures contenus dans le premier flux d'alimentation,
- étant alimentés par le biais d'un bloc brûleur à un foyer dans lequel l'oxydation partielle des hydrocarbures a lieu,
- avec obtention d'un gaz de craquage qui est trempé en aval du foyer par injection d'un milieu de trempe aqueux à une température de 80 à 90 degrés Celsius,
o un flux d'eau de procédé I_{liq}, ainsi que
o un flux de gaz de produit I_{g} étant obtenu, qui
o est refroidi dans une colonne de refroidissement par échange direct de chaleur avec de l'eau de refroidissement, avec obtention
o d'un flux d'eau de procédé II_{liq} en tant que flux de fond,
o d'un flux de gaz de produit II_{g} en tant que flux de tête, ainsi que
o d'un flux latéral, qui
- est appauvri en suie dans un électrofiltre, et un flux d'eau de procédé III_{liq} étant produit dans l'électrofiltre qui est réuni avec les flux d'eau de procédé I_{liq} et II_{liq} pour donner le flux d'eau de procédé IV_{liq}, **caractérisé en ce que** le flux d'eau de procédé IV_{liq} réuni est soumis à une purification par évaporation partielle dans un récipient de détente, le flux d'eau de procédé IV_{liq} réuni étant évaporé en une proportion de 0,01 % en poids à 10 % en poids par rapport au poids total de celui-ci, avec obtention d'un flux d'eau de procédé V_{liq} purifié qui est soutiré au niveau du fond du récipient de détente et qui est conduit sur des canaux de suie comportant des séparateurs de particules de surface avec obtention d'un flux d'eau de procédé VI_{liq} exempt de suie flottante, qui est recyclé dans le procédé.

2. Procédé selon la revendication 1, **caractérisé en ce que** le flux d'eau de procédé V_{liq} purifié est entièrement recyclé dans le procédé.

3. Procédé selon la revendication 1, **caractérisé en ce que** le flux d'eau de procédé VI_{liq} sortant des canaux de suie est divisé et un flux partiel de ce flux d'eau de procédé VI_{liq} est alimenté comme flux d'eau de procédé VII_{liq} dans une tour de refroidissement et est là refroidi et est ensuite renvoyé dans la colonne de refroidissement et le deuxième flux partiel du flux d'eau de procédé VI_{liq} est renvoyé comme flux d'eau de procédé VIII_{liq} dans la zone de trempe en dessous du bloc brûleur.

4. Procédé selon la revendication 1, **caractérisé en ce que** le flux d'eau de procédé VI_{liq} sortant des canaux de suie est divisé et un flux partiel de ce flux d'eau de procédé VI_{liq} est alimenté comme flux d'eau de procédé VII_{iiq} à un échangeur de chaleur et après refroidissement un flux partiel de ce flux d'eau de procédé refroidi est renvoyé dans la colonne de refroidissement et le flux partiel restant est éliminé dans les eaux usées et le deuxième flux partiel du flux d'eau de procédé VI_{liq} sortant des canaux de suie est renvoyé comme flux d'eau de procédé VIII_{liq} dans la zone de trempe en dessous du bloc brûleur.

5. Procédé selon l'une quelconque des revendications 1 à 4, **caractérisé en ce que** le flux d'eau de procédé IV_{liq} réuni est évaporé en une proportion de 0,5 % en poids à 5 % en poids par rapport au poids total de celui-ci.

6. Procédé selon l'une quelconque des revendications 1 à 5, **caractérisé en ce que** l'évaporation partielle est réalisée par détente sous vide.

7. Procédé selon l'une quelconque des revendications 1 à 6, **caractérisé en ce que** l'évaporation partielle est réalisée par détente dans un vide de 50 à 900 mbars a.

8. Procédé selon l'une quelconque des revendications 1 à 7, **caractérisé en ce que** l'évaporation partielle est réalisée par détente dans un vide de 200 à 600 mbars a.

9. Procédé selon l'une quelconque des revendications 1 à 8, **caractérisé en ce que** l'évaporation partielle est réalisée par détente de manière adiabatique.

10. Procédé selon l'une quelconque des revendications 1 à 9, **caractérisé en ce que** l'évaporation partielle est soutenue par un apport de chaleur.

11. Procédé selon la revendication 10, **caractérisé en ce que** l'apport de chaleur est réalisé par alimentation directe de vapeur.
